# FASCICULE DE BREVET EUROPEEN

(11) **EP 3 922 895 B1**
(45) Date de publication et mention de la délivrance du brevet: **22.11.2023**
(21) Numéro de dépôt: 21168522.7
(22) Date de dépôt: 15.04.2021
(51) Int. Cl.: A61M 39/10, B01D 39/08, A61M 39/26, F16L 37/42, F16L 29/02

(54) **ELÉMENT DE FILTRATION D'UN GUIDE-EMBOUT DE PRISE MURALE DE DISTRIBUTION DE FLUIDE**
FILTERELEMENT EINER WANDANSCHLUSSFÜHRUNG FÜR DIE AUSGABE VON FLUIDEN
FILTERING ELEMENT OF A GUIDING END TIP FOR A FLUID DISTRIBUTION WALL SOCKET

(30) Priorité: 10.06.2020 FR 2006069
(43) Date de publication de la demande: 15.12.2021
(73) Titulaire: Air Liquide Medical Systems, 92160 Antony (FR)
(72) Inventeur: CHEVEREAU, Christophe, 92160 Antony (FR); BEAUCHER, Jérôme, 92160 Antony (FR); RUDNIANYN, Philippe, 92160 Antony (FR); FAVRE REGUILLON, Loic, 92160 Antony (FR)
(74) Mandataire: Air Liquide

(56) Documents cités:
- WO-A1-2019/063134
- FR-A1- 2 431 649
- FR-A1- 2 628 820

## Description

L'invention concerne un élément de filtration pour guide-embout de prise de distribution de fluide (i.e. gaz ou vide), et une prise murale de distribution de fluide équipée d'un guide-embout incluant un tel élément de filtration destinée à être utilisée dans un bâtiment hospitalier ou analogue, typiquement une prise murale fixée à une paroi verticale, servant à fournir du gaz ou du vide.

Les prises de distribution de fluide sont utilisées pour distribuer les fluides, en particulier les gaz médicaux (i.e. un gaz pur ou un mélange gazeux) ou le vide (i.e. dépression < 1 atm), au sein des bâtiments hospitaliers ou analogues. Elles sont couramment appelées « prises murales » ou « raccords muraux » car elles sont généralement montées soit directement sur les parois, c'est-à-dire les murs ou analogues, des bâtiments hospitaliers, soit indirectement, par exemple en étant intégrées à un boitier ou analogue qui est lui-même monté sur une paroi, en particulier dans les chambres des patients, dans les salles d'opération ou de soins, notamment les salles de réanimation, ou d'autres pièces

Les prises murales permettent de fournir les fluides médicaux, c'est-à-dire les gaz médicaux véhiculés par les réseaux de canalisations de gaz parcourant les bâtiments hospitaliers, aux appareils et équipements utilisés pour traiter et soigner les patients au sein de ces bâtiments, en particulier les gaz thérapeutiques, tel que l'oxygène, le protoxyde d'azote ou l'air, ou le vide médical (i.e. dépression) permettant d'opérer des aspirations notamment de liquides biologiques, par exemple le sang ou d'autres liquides biologiques.

Ainsi, le document FR-A-2628820 propose une prise de distribution de fluide, appelée raccord à verrouillage automatique, ayant une architecture classique.

Elle comprend un corps de prise de forme allongée comprenant une cavité ou passage central traversant axialement le corps de prise de sorte de relier fluidiquement une extrémité amont, aussi appelée extrémité d'entrée ou extrémité distale, à une extrémité aval, aussi appelée extrémité de sortie ou extrémité proximale comprenant un orifice de fourniture de fluide, c'est-à-dire de gaz ou de vide. Le fluide « circule » dans la prise, lorsqu'un connecteur d'un appareil ou équipement médical, notamment le connecteur d'une conduite de gaz, est raccordé mécaniquement et fluidiquement à la prise.

Des éléments de contrôle du passage de fluide internes à la prise murale permettent de contrôler la libération du gaz, en particulier d'empêcher toute délivrance du gaz quand aucun connecteur s'y est branché. Ces éléments de contrôle du passage de fluide comprennent un guide-embout extractible agencé du côté proximal de la prise, c'est-à-dire vers l'avant de la prise, et une chambre à bille-clapet extractible, du côté distal de la prise, c'est-à-dire vers l'arrière ou le fond de la prise, qui sert à empêcher toute circulation de fluide lorsque le guide-embout est extrait du corps de prise, ou à limiter le débit de fuite.

Le guide-embout comprend un clapet de tête coulissant et l'orifice de sortie de gaz ou d'aspiration pour le vide. Le clapet de tête coulissant coopère avec un siège de clapet pour assurer ou, à l'inverse, interrompre l'étanchéité fluidique entre eux et ainsi empêcher ou, à l'inverse, autoriser le passage de fluide, i.e. gaz ou vide, lorsqu'un connecteur d'un appareil ou équipement médical est raccordé à la prise murale. Un élément élastique, tel un ressort, agit sur le clapet de tête coulissant pour le repousser en direction du siège de clapet.

Le guide-embout comprend ou est formé habituellement deux parties, à savoir une partie arrière et une partie avant, fixées l'une à l'autre, par exemple par vissage.

La partie avant ou « tête » du guide-embout comprend des moyens de fixation dudit guide-embout au sein de la prise de distribution de fluide, par exemple un filetage périphérique externe, et est par ailleurs traversé par un passage axial en communication fluidique avec l'orifice de la partie arrière du guide-embout qui communique fluidiquement avec le logement interne de la partie arrière du guide-embout.

La partie arrière ou « corps » de guide-embout comprend une paroi périphérique délimitant un logement interne, i.e. un volume interne ou lumen, s'étendant entre une extrémité ouverte et une extrémité borgne. L'extrémité ouverte porte un orifice communiquant avec le logement interne, alors que la paroi périphérique comprenant une ou plusieurs ouvertures latérales de passage de gaz communiquant avec le logement interne. Le fluide circule au travers des ouvertures latérales et/ou de l'orifice pour entrer ou sortir du logement interne. Le clapet de tête coulissant est agencé mobile dans le logement interne.

Ce type de prise murale doit subir régulièrement des opérations de maintenance, notamment afin de garantir leur bon fonctionnement, de remplacer les éléments usés ou détériorés (e.g. utilisation intensive), de les nettoyer.... Lors de ces opérations, il faut pouvoir démonter puis remonter facilement et rapidement les différents éléments internes de la prise murale, en particulier le guide-embout situé vers l'avant du corps de prise et la chambre à bille-clapet. Il doit aussi être possible de remplacer uniquement les éléments usés ou détériorés du guide-embout, sans qu'il ne soit besoin de les remplacer tous, en particulier ceux qui ne sont pas usés ou détériorés.

Enfin, il faut également assurer une filtration du fluide (i.e. gaz ou vide) traversant la prise murale afin de garantir qu'il soit exempt de poussières ou autres contaminants/polluants solides.

On connait par ailleurs WO2019/063134 qui enseigne un élément de filtre pour outil à eau, qui est de forme cylindrique, et FR-A-2431649 qui divulgue une prise murale de distribution de gaz incorporant un ou plusieurs filtres internes.

Le problème est de pouvoir améliorer la structure du guide-embout d'une prise de distribution de fluide (i.e. gaz ou vide), en particulier la partie arrière du guide-embout, de manière à pouvoir répondre aux différentes exigences et/ou problématiques susmentionnées.

La solution concerne alors un élément de filtration pour un guide-embout d'une prise de distribution de fluide, i.e. gaz ou vide, comprenant une pièce-support comprenant une paroi périphérique délimitant un logement interne et s'étendant entre une extrémité ouverte comprenant un orifice et une extrémité borgne, l'orifice communiquant avec le logement interne, et la paroi périphérique comprenant en outre au moins une ouverture latérale communiquant avec le logement interne, caractérisé en ce que la pièce-support comprend plusieurs ouvertures latérales, chaque ouverture latérale comprenant une membrane de filtration de gaz agencée dans ladite ouverture latérale de manière à recouvrir ladite ouverture latérale.

Selon le mode de réalisation considéré, l'élément de filtration de l'invention peut comprendre l'une ou plusieurs des caractéristiques suivantes :
- l'élément de filtration forme la partie arrière ou corps d'un guide-embout d'une prise de distribution de fluide.
- la pièce-support a une forme générale tubulaire à fond borgne, c'est-à-dire fermée à une extrémité.
- l'extrémité ouverte de la pièce-support comprend un orifice au travers duquel le fluide (i.e. gaz ou vide) peut circuler, c'est-à-dire entrer ou sortir de l'élément de filtration.
- le logement interne de la pièce-support a une forme cylindrique.
- la pièce-support comprend de 2 à 6 ouvertures latérales, de préférence de 2 à 4 ouvertures latérales.
- les ouvertures latérales sont préférentiellement identiques les unes aux autres ou, selon un autre mode de réalisation, elles peuvent avoir des formes différentes les uns des autres.
- les membranes de filtration recouvrent complètement les ouvertures latérales dans lesquelles elles sont agencées, c'est-à-dire que les sections de passage des ouvertures latérales sont totalement obturées par les membranes de filtration.
- les membranes de filtration sont conçues pour filtrer le fluide (i.e. gaz ou vide) qui les traversent.
- les membranes de filtration sont formées d'une pièce unique de forme cylindrique.
- la pièce cylindrique portant les membranes de filtration a un diamètre inférieur ou égal, de préférence approximativement égal, au diamètre interne du logement interne de la pièce-support.
- selon un mode de réalisation, la pièce cylindrique portant les membranes de filtration est insérée et non fixée (i.e. libre) dans le logement interne de la pièce-support.
- selon un autre mode de réalisation, la pièce cylindrique portant les membranes de filtration est insérée et fixée (i.e. libre) dans le logement interne de la pièce-support.
- les membranes de filtration sont fixées par collage, thermo-soudage, surmoulage ou autre, de préférence par surmoulage.
- les membranes de filtration sont formées d'une toile ou tissu (i.e. matériau tissé) en polymère.
- les membranes de filtration comprennent des pores (aussi appelée mailles) ayant une taille comprise entre 50 et 300 µm.
- les membranes de filtration comprennent des pores (aussi appelée mailles) compris entre 50 et 80 µm, en particulier lorsqu'elles sont destinées à filtrer un gaz, notamment un gaz médical.
- les membranes de filtration comprennent des pores (aussi appelée mailles) compris entre 200 et 300 µm, en particulier lorsqu'elles sont destinées à filtrer du vide ou de l'air-instrument.
- les membranes de filtration sont formées d'une pièce unique de forme cylindrique formée d'une toile ou d'un tissu en polymère.
- la pièce-support est surmoulée autour de la pièce unique de forme cylindrique formée d'une toile ou d'un tissu en polymère.
- les membranes de filtration sont formées d'une pièce en polymère de forme cylindrique autour de laquelle est surmoulée la pièce-support.
- la pièce-support comprend un axe longitudinal (XX), c'est-à-dire qu'elle s'étend axialement selon l'axe longitudinal (XX).
- la pièce-support est une pièce de révolution.
- chaque membrane de filtration a une épaisseur inférieure à l'épaisseur de la paroi périphérique autour de l'ouvertures latérale dans laquelle elle est agencée.
- les membranes de filtration sont préférentiellement en polymère, par exemple en polyamide, tel du Nylon^{®}.
- les membranes de filtration sont, selon un autre mode de réalisation, en métal, par exemple de l'acier inoxydable.
- la pièce-support est en polymère, par exemple en polyamide, ou en métal ou alliage métallique, par exemple laiton ou acier inoxydable.
- la pièce-support a une forme générale cylindrique.
- les ouvertures latérales sont de section carrée ou rectangulaire, ou autre, par exemple ovale ou ellipsoïdale.
- la pièce-support comprend plusieurs tronçons successifs de diamètres externes différents, i.e. des tronçons cylindriques ou approximativement cylindriques.
- la pièce-support comprend un tronçon amont à son extrémité ouverte ; un tronçon aval à son extrémité borgne ; et au moins un tronçon intermédiaire agencé entre les tronçons amont et aval, ledit tronçon intermédiaire comprenant les ouvertures.
- les tronçons amont et aval sont cylindriques.
- le tronçon intermédiaire est cylindrique ou approximativement cylindrique.
- le diamètre externe du tronçon intermédiaire comprenant les ouvertures est inférieur aux diamètres externes des tronçons amont et aval.
- les tronçons amont et aval sont reliés l'un à l'autre par des éléments de paroi longilignes formant tout ou partie du tronçon intermédiaire.
- les éléments de paroi longilignes sont parallèles les uns aux autres et à l'axe longitudinal (XX).
- les ouvertures du tronçon intermédiaire sont bordées par les tronçons amont et aval, et les éléments de paroi longilignes.
- les tronçons amont et aval, et les éléments de paroi longilignes sont formés d'une seule pièce, par exemple par moulage.
- les tronçons amont et aval, et les éléments de paroi longilignes sont formés de matériau polymère, i.e. sont en plastique.
- le diamètre externe du tronçon amont est par exemple compris entre 12 et 16 mm.
- le diamètre externe du tronçon aval est par exemple compris entre 11 et 15 mm.
- le diamètre externe du tronçon cylindrique intermédiaire est par exemple compris entre 11 et 15 mm.
- l'extrémité borgne de la pièce-support comprend une tige-poussoir faisant saillie axialement (axe XX) au centre de la face externe de l'extrémité borgne.
- la pièce-support a une longueur de l'ordre de 20 à 25 mm.
- le logement interne de la pièce-support a un diamètre interne de l'ordre de 9 à 12 mm.
- l'extrémité ouverte de la pièce-support comprend un taraudage permettant de la fixer à la partie avant d'un guide-embout.

L'invention concerne aussi un guide-embout pour prise de distribution de fluide comprenant une partie avant, aussi appelée tête, et une partie arrière, aussi appelée corps, fixées l'une à l'autre, caractérisé en ce qu'un élément de filtration selon l'invention forme au moins une partie de la partie arrière du guide-embout.

Selon le mode de réalisation considéré, le guide-embout de l'invention peut comprendre l'une ou plusieurs des caractéristiques suivantes :
- un clapet de tête coulissant est agencé dans le logement interne de l'élément de filtration.
- un moyen élastique, tel un ressort, est agencé dans le logement interne de l'élément de filtration.
- le moyen élastique est agencé pour agir sur, i.e. repousser, le clapet de tête en direction d'un siège de clapet.
- la partie avant du guide-embout comprend une gorge périphérique annulaire et un élément de joint agencé dans ladite gorge périphérique annulaire, en particulier un joint torique.
- la partie avant du guide-embout comprend en outre un filetage périphérique externe.
- la partie avant du guide-embout comprend en outre un passage axial de fluide en communication fluidique avec le logement interne de l'élément de filtration, via l'orifice de l'élément de filtration.
- la partie avant du guide-embout comprend le siège de clapet coopérant avec le clapet de tête.

L'invention concerne aussi une prise de distribution de fluide, c'est-à-dire de gaz ou de vide (i.e. dépression), comprenant un corps de prise dans lequel est agencé un élément de filtration de gaz selon l'invention.

Selon le mode de réalisation considéré, la prise de distribution de fluide de l'invention peut comprendre l'une ou plusieurs des caractéristiques suivantes :
- le corps de prise comprend en outre une chambre à bille contenant une bille-clapet coopérant avec la tige-poussoir de la pièce-support.
- la chambre à bille est extractible.
- la chambre à bille est visée dans le corps de prise, en particulier dans le passage central du corps de prise.
- elle est une prise murale, c'est-à-dire qu'elle comprend des moyens de fixation permettant de la fixer à une paroi, tel un mur ou analogue.
- l'élément de filtration de gaz est monté de manière amovible, c'est-à-dire démontable, dans le corps de prise.
- elle est reliée à une canalisation de gaz ou de vide, en particulier au réseau de canalisations de gaz ou de vide (i.e. dépression) d'un bâtiment hospitalier.

L'invention concerne en outre une utilisation d'une prise de distribution de fluide selon l'invention pour distribuer, i.e. fournir, un gaz ou mélange gazeux, ou du vide (i.e. aspiration à une pression < 1 atm) au sein d'un bâtiment hospitalier.

Le gaz ou mélange gazeux peut être de l'oxygène, de l'air, un mélange N₂O/O₂.... ou autre.

La prise de distribution de fluide selon l'invention est reliée fluidiquement au réseau de canalisation du bâtiment hospitalier.

L'invention va maintenant être mieux comprise grâce à la description détaillée suivante, faite à titre illustratif mais non limitatif, en référence aux figures annexées parmi lesquelles :
Fig. 1 est une vue latérale d'un élément de filtration de gaz formant la partie arrière d'un guide-embout de prise de distribution de fluide selon l'invention,
Fig. 2 est une vue latérale schématique d'un guide-embout comprenant un élément de filtration de gaz selon l'invention formant la partie arrière dudit guide-embout, et
Fig. 3 est une vue en coupe d'une prise de distribution de gaz incluant un guide-embout comprenant un élément de filtration de gaz selon l'invention.

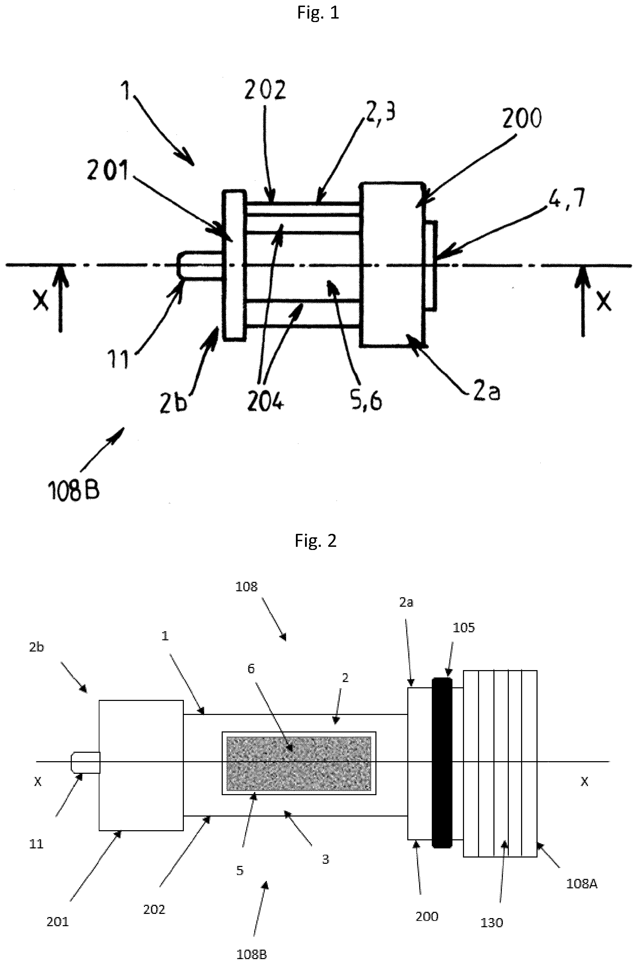

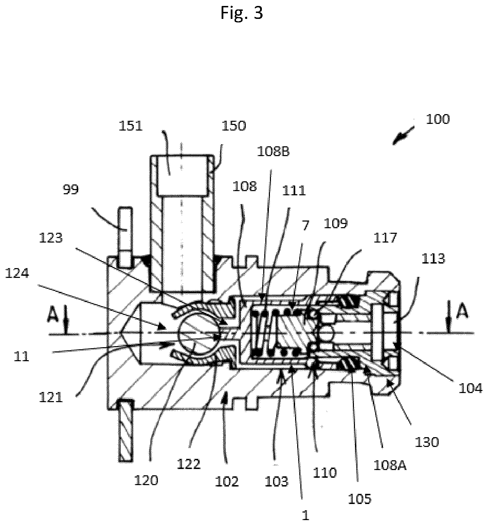

Fig. 3 représente un mode de réalisation d'une prise de distribution 100 de gaz selon l'invention, par exemple d'oxygène ou d'air médical, comprenant un corps de prise 102 de forme allongée, selon un axe longitudinal AA, lequel est traversé axialement par un passage central 103 formant un logement interne au sein duquel sont agencés des éléments de contrôle du passage de gaz, en particulier le guide-embout 108 de Fig. 2 et la chambre à bille 122, comme expliqué ci-après. Une telle architecture est classique.

Le guide-embout 108 extractible est formé de deux parties principales fixées l'une à l'autre par exemple par vissage, à savoir une partie avant 108A ou « tête » de guide-embout 108 et une partie arrière 108B ou « corps » de guide-embout 108 comprenant l'élément de filtration de gaz 1 selon l'invention, comme illustré sur les Fig. 1 et Fig. 2 et détaillé ci-après.

La partie avant 108A comprend des moyens de fixation 130 du guide-embout 108 au sein de la prise de distribution de fluide 100, à savoir ici un filetage périphérique externe visible sur la Fig. 2 venant coopérer avec un taraudage aménagé dans le corps 102 de prise 100, et est par ailleurs traversé par un passage axial comprenant un orifice proximal 104.

Dans le cas de la prise 100 de Fig. 3, le gaz est distribué, c'est-à-dire sort de la prise 100, par l'orifice proximal 104 lorsqu'un appareil, dispositif ou équipement utilisant ou véhiculant le gaz y est raccordé via un connecteur adapté, tel un conduit flexible amenant le gaz à un appareil de respiration assisté, tel un ventilateur médical. L'orifice proximal 104 est de section circulaire. A l'inverse, lorsque la prise 100 est une prise de vide, c'est par cet orifice proximal 104 que s'opère l'aspiration du gaz, c'est-à-dire que s'applique la dépression, permettent d'aspirer les liquides biologiques ou autres.

Par ailleurs, la partie arrière 108B du guide-embout 108 comprend l'élément de filtration de gaz 1 selon l'invention, comme illustré en Fig. 2.

Comme détaillé en Fig. 1 et Fig. 2, l'élément de filtration de gaz 1 de l'invention, formant tout ou partie de la partie arrière 108B du guide-embout 108, comprend une pièce-support 2 avec une paroi périphérique 3 délimitant un logement interne 7 ou lumen.

La pièce-support 2 est de forme allongée, c'est-à-dire tubulaire. Elle s'étend entre une extrémité ouverte 2a comprenant un orifice 4 et une extrémité borgne 2b. L'orifice 4 communique avec le logement interne 7. La paroi périphérique 3 comprenant en outre des ouvertures latérales 5, par exemple de 2 à 4 ouvertures latérales, ici de forme rectangulaire, communiquant avec le logement interne 7. Une membrane de filtration 6 de gaz est agencée dans chacune des ouvertures latérales 5 de manière à recouvrir les ouvertures latérales 5 de sorte que le gaz passant par ces ouvertures latérales 5 soit filtré par les membranes de filtration 6 qui y sont disposées. Le fluide, i.e. gaz ou vide, circule au travers des ouvertures latérales 5 et de l'orifice 4 pour entrer ou sortir du logement interne 7.

Un clapet de tête 109 coulissant est agencé mobile selon l'axe AA dans le logement interne 7, i.e. coulissant en translation, au sein dudit logement axial 7.

Un élément élastique 111, tel un ressort cylindrique ou analogue, est agencé dans le logement axial 7 de l'élément de filtration 1 du guide-embout 108, de préférence dans le fond borne et autour de la portion arrière du clapet de tête 109, lequel élément élastique 111 vient prendre appui, d'une part, sur le fond borgne de l'élément de filtration 1 et, d'autre part, sur un épaulement annulaire solidaire du clapet de tête 109, par exemple formé dans la paroi périphérique externe du clapet de tête 109. L'élément élastique 111 permet de normalement repousser le clapet de tête 109 contre un siège de clapet 110 aménagé dans le guide-embout 108, par exemple au sein de la paroi interne du guide-embout 108, de sorte de contrôler le passage de fluide dans le corps de prise 102.

Par ailleurs, le guide-embout 108 comprend aussi un élément d'étanchéité 105 permettant d'assurer une étanchéité fluidique entre ledit guide-embout 108 et le corps de prise 102.

Un autre élément d'étanchéité 117, porté par le clapet 109, permet quant à lui d'assurer une étanchéité fluidique entre le clapet 109 et le siège de clapet 110, lorsque le clapet de tête 109 est repoussé contre le siège de clapet 110 par l'élément élastique 111.

Par ailleurs, la prise 100 de distribution de fluide comprend aussi un système de sécurité agencé au fond du passage central 103, c'est-à-dire au fond du corps de prise 102, permettant d'empêcher ou limiter la circulation de gaz en direction de l'orifice de sortie 104, lorsque le guide-embout 108 est extrait du corps de prise 102, par exemple lors d'une opération de maintenance du guide-embout 108. Ce système de sécurité, appelé chambre à bille-clapet 122, comprend un compartiment 121, i.e. une chambre, dans lequel est logée une bille-clapet 120, à savoir ici une bille de forme sphérique, coopérant avec un siège de bille-clapet situé dans le compartiment 121 de la chambre à bille-clapet 122.

La chambre à bille-clapet 122 est aussi extractible du corps de prise 102 afin de permettre sa maintenance. Elle permet d'empêcher que le gaz ne puisse s'échapper de la prise 100 ou que l'aspiration par le vide (i.e. dépression) ne se fasse, c'est-à-dire que de l'air ne puisse entrer dans le réseau de vide relié à la prise 100 et y faire remonter la pression (< 1 atm) qui y règne, lorsque les éléments de contrôle du passage de gaz, typiquement le guide-embout 108, sont démontés et extraits du corps de prise 102, notamment lors d'une opération de vérification, de maintenance ou de remplacement.

La bille-clapet 120 fait office de clapet de sécurité venant, sous l'effet de la pression fluidique ou de la dépression (i.e. vide) s'exerçant sur la bille-clapet 120 en cas de démontage du guide-embout 108, appuyer sur et obturer un siège de clapet situé dans le compartiment 121. A l'inverse, quand les éléments de contrôle du passage de gaz sont montés dans le corps de prise 102, le guide-embout 108 vient appuyer sur la bille-clapet 120 pour la décoller du siège de clapet et autoriser ainsi la communication fluidique au travers du canal de liaison 123 reliant fluidiquement le compartiment 121 au passage axial 103 du corps 102 de la prise 100. Ceci peut se faire via une tige-poussoir 11, c'est-à-dire une tige ou expansion axiale, faisant saillie sur la surface externe arrière du guide-embout 8 et solidaire de celui-ci, qui est orientée de sorte de traverser axialement le canal de liaison 123 pour venir appuyer sur la bille-clapet 120 et ainsi la décoller de son siège. La tige axiale 11 est portée par la pièce-support 2 de l'élément de filtration 1 de l'invention, comme visible sur les Fig. 1 à Fig. 3, et détaillé ci-après.

La prise de distribution 1 de fluide de FIG. 3 peut être montée soit directement sur une paroi (non montrée), c'est-à-dire un mur ou analogue, d'un bâtiment hospitalier au moyen d'un étrier de fixation 99, soit être intégrées à un boitier ou analogue qui est lui-même monté sur une paroi. Elle permet de fournir le vide ou les gaz médicaux véhiculés par le réseau de canalisations de fluide parcourant le bâtiment hospitalier, aux appareils et équipements utilisés pour traiter et soigner les patients au sein de ces bâtiments. Pour ce faire, elles peuvent être raccordées au réseau de canalisations de fluide d'un tel bâtiment, via une tubulure arrière 150, tel un raccord ou analogue, en communication fluidique, via son lumen 151, avec, d'une part, ledit réseau de canalisations de fluide et, d'autre part, avec le passage central interne de la prise 100 via la chambre à bille 122 qui communique avec le lumen 151 de la tubulure arrière 150, via une ouverture large 124 de la chambre à bille 122.

La partie arrière 108B du guide-embout 108 comprend une partie détachable, à savoir l'élément de filtration 1 selon l'invention, qui est illustré sur la Fig. 1, lequel vient se fixer, par exemple par vissage, à la partie avant 108A du guide-embout 108, comme schématisé en Fig. 2.

L'élément de filtration 1 selon l'invention comprend, comme visible sur Fig. 1 et Fig. 2, une pièce-support 2, formant un corps tubulaire allongé, comprenant plusieurs tronçons successifs 200-202 ayant des diamètres externes différents, à savoir un tronçon amont 200 à son extrémité ouverte 2a, un tronçon aval 201 à son extrémité borgne 2b, et un tronçon intermédiaire 202 agencé entre les tronçons amont et aval 200, 201, lequel porte les ouvertures 5 obturées par les membranes de filtration 6.

Les tronçons amont 200 et aval 201 sont cylindriques et ont des diamètres externes égaux ou différents, par exemple compris entre 11 et 16 mm, alors que le tronçon intermédiaire 202 est cylindrique ou approximativement cylindrique, et a un diamètre inférieur au ou aux diamètres externes des tronçons amont 200 et aval 201, par exemple compris entre 11 et 15 mm. La pièce-support 2 a par exemple une longueur de l'ordre de 20 à 25 mm.

Les ouvertures 5 sont ici de forme rectangulaire mais, bien entendu, elles pourraient être d'une autre forme, par exemple de forme carrée, ovale, ellipsoïdale ou autre. Les membranes de filtration 6 ont des formes complémentaires de celles des ouvertures 5 de manière à les recouvrir complètement de sorte que tout le gaz passant au travers des ouvertures 5 soit filtré par ces membranes de filtration 6.

Dans le mode de réalisation de la Fig. 1, les tronçons amont 200 et aval 201 sont des cylindres reliés l'un à l'autre par des éléments de paroi longilignes 203 formant tout ou partie du tronçon intermédiaire 202. Ces éléments de paroi longilignes 204 sont parallèles les uns aux autres et à l'axe longitudinal (XX). Les ouvertures 5 sont donc bordées par les tronçons amont 200 et aval 201, et les éléments de paroi longilignes 204.

Avantageusement, les tronçons amont 200 et aval 201, et les éléments de paroi longilignes 204 sont formés d'une seule pièce, par exemple en polymère, i.e. plastique, ou en métal ou alliage métallique, par exemple par moulage ou décolletage.

Comme on le voit sur la Fig. 1, l'extrémité borgne 2b de la pièce-support 2 porte, sur face externe, la tige-poussoir 11 qui coopère avec la bille-clapet 120, comme expliqué ci-avant. La tige-poussoir 11 fait saillie axialement (selon l'axe XX) au centre de la face externe de l'extrémité borgne de la pièce-support 2.

Le logement interne 7 de la pièce-support 2 a un diamètre interne de l'ordre de 9 à 12 mm de manière à pouvoir accueillir le clapet de tête 109 et le ressort 111, comme expliqué ci-avant.

Afin de permettre de fixer l'élément de filtration 1 selon l'invention formant la partie arrière 108B du guide-embout 108, à la partie avant 108A du guide-embout 108, l'extrémité ouverte 2a de la pièce-support 2 comprend un taraudage (non visible) ou tout autre système de fixation adapté, permettant de fixer l'élément de filtration 1 à la partie avant 108A du guide-embout 108.

Les membranes de filtration 6 agencées dans les ouvertures 5 sont conçues pour filtrer le fluide, i.e. gaz ou vide, qui les traversent. Elles peuvent être en polymère ou en métal. Elles ont des pores ayant des dimensions inférieures aux particules qu'elles doivent arrêter, telles des poussières, des débris, des résidus, des bactéries ou toutes autres particules, par exemple des pores de diamètre compris entre 50 et 80 µm, lorsqu'elles sont destinées à filtrer un gaz, notamment un gaz médical (air médical, oxygène...), ou compris entre 200 et 300 µm, lorsqu'elles sont destinées à filtrer du vide (i.e. dépression) ou de l'air-instrument.

Les membranes de filtration 6 sont constituées ou portées par une pièce unique de forme cylindrique préférentiellement formée d'une toile de polyamide, tel du Nylon^{®}, autour de laquelle est surmoulé le reste de la pièce support 2, par exemple une toile en polyamide de référence SEFAR-NITEXO commercialisée par la société Sefar.

Fig. 3 représente un mode de réalisation d'une prise 100 de distribution de gaz. Son architecture globale est très similaire à celle d'une prise de distribution de vide, c'est-à-dire de dépression. Une différence majeure réside toutefois dans le fait que, dans une prise de distribution de vide, l'aspiration du vide (i.e. dépression), c'est-à-dire la circulation du gaz aspiré au travers de la prise, se fait dans le sens opposé à celui de la circulation de gaz au sein de la prise 100 de Fig. 3. Ainsi, le gaz (e.g. air) est aspiré par la dépression régnant dans le corps de prise 102 par l'orifice proximal 104, ce qui permet d'obtenir un effet de succion, c'est-à-dire d'aspiration, en amont de la prise 100, c'est-à-dire dans un dispositif médical (non montré) raccordé fluidiquement à la prise 100. L'orifice proximal 104 sert donc à aspirer du gaz et non pas à en distribuer. Une conséquence de cette circulation inverse du fluide est que, dans une prise 100 de distribution de vide, la chambre à bille est orientée à l'inverse de celle de la Fig. 3 et que par ailleurs, la bille-clapet n'est pas complètement sphérique mais comprend par exemple uniquement une tête hémisphérique sur laquelle vient appuyer la tige 111 du guide-embout 108 au travers du canal de liaison 122, comme expliqué ci-avant.

D'une façon générale, les prises 1 de l'invention sont généralement agencées sur des parois, tels des murs ou analogues, des bâtiments hospitaliers et sont conçues pour permettre de fournir soit des gaz thérapeutiques, i.e. gaz médicaux, en particulier de l'oxygène, du protoxyde d'azote, de l'air ou tout autre gaz ou mélange gazeux, soit du vide médical (i.e. une dépression) servant à opérer des aspirations notamment de liquides biologiques, par exemple le sang ou d'autres liquides biologiques.

## Revendications

1. Elément de filtration (1) pour guide-embout (108) de prise de distribution de fluide (100) comprenant une pièce-support (2) comprenant une paroi périphérique (3) délimitant un logement interne (7) et s'étendant entre une extrémité ouverte (2a) comprenant un orifice (4) et une extrémité borgne (2b), l'orifice (4) communiquant avec le logement interne (7), et la paroi périphérique (3) comprenant en outre au moins une ouverture latérale (5) communiquant avec le logement interne (7), **caractérisé en ce que** la pièce-support (2) comprend plusieurs ouvertures latérales (5), chaque ouverture latérale (5) comprenant une membrane de filtration (6) de gaz agencée dans ladite ouverture latérale (5) de manière à recouvrir ladite ouverture latérale (5).

2. Elément selon la revendication 1, **caractérisé en ce que** la pièce-support (2) comprend de 2 à 4 ouvertures latérales (5).

3. Elément selon la revendication 1, **caractérisé en ce que** les membranes de filtration (6) sont formées d'une pièce unique de forme cylindrique.

4. Elément selon la revendication 3, **caractérisé en ce que** la pièce unique de forme cylindrique est un tissu polymère.

5. Elément selon l'une des revendications 3 ou 4, **caractérisé en ce que** les membranes de filtration (6) sont formées d'une pièce cylindrique autour de laquelle est surmoulée la pièce-support (2).

6. Elément selon la revendication 4 ou 5, **caractérisé en ce que** le tissu est en polyamide ou la pièce-support (2) est en polymère.

7. Elément selon l'une des revendications 1, 5 ou 6, **caractérisé en ce que** la pièce-support (2) comprend plusieurs tronçons successifs (200-202) comprenant :
- un tronçon amont (200) à son extrémité ouverte (2a),
- un tronçon aval (201) à son extrémité borgne (2b), et
- un tronçon intermédiaire (202) agencé entre les tronçons amont et aval (200, 201), ledit premier tronçon intermédiaire (2002) comprenant les ouvertures (5).

8. Elément selon l'une des revendications 1 ou 7, **caractérisé en ce que** l'extrémité borgne (2b) de la pièce-support (2) comprend une tige-poussoir (11) faisant saillie axialement (axe XX) au centre de la face externe de l'extrémité borgne (2b).

9. Elément selon l'une des revendications 1 ou 7, **caractérisé en ce que** l'extrémité ouverte (2a) de la pièce-support (2) comprend un taraudage interne.

10. Guide-embout (108) pour prise de distribution de fluide (100) comprenant une partie avant (108A) et une partie arrière (108B) fixées l'une à l'autre, **caractérisé en ce qu'**un élément de filtration (1) selon l'une des revendications précédentes forme au moins une partie de la partie arrière (108B) dudit guide-embout (108).

11. Guide-embout selon la revendication 10, **caractérisé en ce qu'**un clapet de tête coulissant (109) et un moyen élastique (111) sont agencés dans le logement interne (7) de l'élément de filtration (1).

12. Prise de distribution de fluide (100) comprenant un corps de prise (102) dans lequel est agencé un guide-embout (108) selon l'une des revendications 10 ou 11.

13. Prise de distribution de fluide selon la revendication 12, **caractérisée en ce que** le corps de prise (102) comprend en outre une chambre à bille (122) contenant une bille-clapet (120) coopérant avec la tige-poussoir (11) de la pièce-support (2).

14. Utilisation d'une prise de distribution de fluide (100) selon l'une des revendications 12 ou 13 pour distribuer un gaz ou mélange gazeux, ou du vide au sein d'un bâtiment hospitalier.

## Patentansprüche

1. Filterelement (1) für ein Führungsendstück (108) für einen Fluidverteilungsanschluss (100), umfassend ein Stützteil (2), das eine Umfangswand (3) umfasst, die eine innere Aufnahme (7) begrenzt und sich zwischen einem offenen Ende (2a), das eine Bohrung (4) umfasst, und einem geschlossenen Ende (2b) erstreckt, wobei die Bohrung (4) mit der inneren Aufnahme (7) in Verbindung steht, und wobei die Umfangswand (3) ferner mindestens eine seitliche Öffnung (5) umfasst, die mit der inneren Aufnahme (7) in Verbindung steht, **dadurch gekennzeichnet, dass** das Stützteil (2) mehrere seitliche Öffnungen (5) umfasst, wobei jede seitliche Öffnung (5) eine Gasfiltermembran (6) umfasst, die in der seitlichen Öffnung (5) so angeordnet ist, dass sie die seitliche Öffnung (5) abdeckt.

2. Element nach Anspruch 1, **dadurch gekennzeichnet, dass** das Stützteil (2) zwei bis vier seitliche Öffnungen (5) umfasst.

3. Element nach Anspruch 1, **dadurch gekennzeichnet, dass** die Filtermembranen (6) aus einem einzigen Stück von zylindrischer Form gebildet sind.

4. Element nach Anspruch 3, **dadurch gekennzeichnet, dass** das einzige Stück von zylindrischer Form ein Polymergewebe ist.

5. Element nach einem der Ansprüche 3 oder 4, **dadurch gekennzeichnet, dass** die Filtermembranen (6) aus einem zylindrischen Stück gebildet sind, um das herum das Stützteil (2) angeformt ist.

6. Element nach Anspruch 4 oder 5, **dadurch gekennzeichnet, dass** das Gewebe aus Polyamid ist oder das Stützteil (2) aus Polymer ist.

7. Element nach einem der Ansprüche 1, 5 oder 6, **dadurch gekennzeichnet, dass** das Stützteil (2) mehrere aufeinanderfolgende Abschnitte (200-202) umfasst, umfassend:
- einen stromaufwärtigen Abschnitt (200) an seinem offenen Ende (2a),
- einen stromabwärtigen Abschnitt (201) an seinem geschlossenen Ende (2b) und
- einen Zwischenabschnitt (202), der zwischen den stromaufwärtigen und stromabwärtigen Abschnitten (200, 201) angeordnet ist, wobei der erste Zwischenabschnitt (2002) die Öffnungen (5) umfasst.

8. Element nach einem der Ansprüche 1 oder 7, **dadurch gekennzeichnet, dass** das geschlossene Ende (2b) des Stützteils (2) eine Stößelstange (11) umfasst, die im Zentrum der Außenseite des geschlossenen Endes (2b) axial (Achse XX) vorspringt.

9. Element nach einem der Ansprüche 1 oder 7, **dadurch gekennzeichnet, dass** das offene Ende (2a) des Stützteils (2) ein Innengewinde umfasst.

10. Führungsendstück (108) für einen Fluidverteilungsanschluss (100), umfassend einen vorderen Teil (108A) und einen hinteren Teil (108B), die aneinander befestigt sind, **dadurch gekennzeichnet, dass** ein Filterelement (1) nach einem der vorhergehenden Ansprüche mindestens einen Teil des hinteren Teils (108B) des Führungsendstücks (108) bildet.

11. Führungsendstück nach Anspruch 10, **dadurch gekennzeichnet, dass** ein gleitendes Kopfventil (109) und ein elastisches Mittel (111) in der inneren Aufnahme (7) des Filterelements (1) angeordnet sind.

12. Fluidverteilungsanschluss (100), umfassend einen Anschlusskörper (102), in dem ein Führungsendstück (108) nach einem der Ansprüche 10 oder 11 angeordnet ist.

13. Fluidverteilungsanschluss nach Anspruch 12, **dadurch gekennzeichnet, dass** der Anschlusskörper (102) ferner eine Kugelkammer (122) umfasst, die eine Ventilkugel (120) enthält, die mit der Stößelstange (11) des Stützteils (2) zusammenwirkt.

14. Verwendung eines Fluidverteilungsanschlusses (100) nach einem der Ansprüche 12 oder 13 zum Ausgeben eines Gases oder Gasgemisches oder von Vakuum in einem Krankenhausgebäude.

## Claims

1. Filtration element (1) for an endpiece guide (108) of a fluid distribution outlet (100) comprising a support piece (2) comprising a peripheral wall (3) delimiting an internal housing (7) and extending between an open end (2a) comprising an orifice (4) and a blind end (2b), the orifice (4) communicating with the internal housing (7) and the peripheral wall (3) further comprising at least one lateral opening (5) communicating with the internal housing (7), **characterized in that** the support piece (2) comprises several lateral openings (5), each lateral opening (5) comprising a gas filtering membrane (6) arranged in said lateral opening (5) in such a way as to cover said lateral opening (5).

2. Element according to Claim 1, **characterized in that** the support piece (2) comprises from two to four lateral openings (5).

3. Element according to Claim 1, **characterized in that** the filtering membranes (6) are formed as a cylinder-shaped single piece.

4. Element according to Claim 3, **characterized in that** the cylinder-shaped single piece is a woven polymer fabric.

5. Element according to either of Claims 3 and 4, **characterized in that** the filtering membranes (6) are formed as a cylindrical piece around which the support piece (2) is overmoulded.

6. Element according to Claim 4 or 5, **characterized in that** the woven fabric is made of polyamide or the support piece (2) is made of polymer.

7. Element according to one of Claims 1, 5 and 6, **characterized in that** the support piece (2) comprises several successive portions (200-202) comprising:
- an upstream portion (200) at its open end (2a),
- a downstream portion (201) at its blind end (2b), and
- an intermediate portion (202) positioned between the upstream and downstream portions (200, 201), said intermediate portion (2002) comprising the openings (5) .

8. Element according to either of Claims 1 and 7, **characterized in that** the blind end (2b) of the support piece (2) comprises a pushrod (11) projecting axially (axis XX) at the centre of the external face of the blind end (2b).

9. Element according to either of Claims 1 and 7, **characterized in that** the open end (2a) of the support piece (2) has a tapped internal thread.

10. Endpiece guide (108) for a fluid distribution outlet (100) comprising a front part (108A) and a rear part (108B) fixed to one another, **characterized in that** a filtering element (1) according to one of the preceding claims forms at least part of the rear part (108B) of said endpiece guide (108).

11. Endpiece guide according to Claim 10, **characterized in that** a sliding head valve (109) and an elastic means (111) are arranged in the internal housing (7) of the filtering element (1).

12. Fluid distribution outlet (100) comprising an outlet body (102) in which there is arranged an endpiece guide (108) according to either of Claims 10 and 11.

13. Fluid distribution outlet according to Claim 12, **characterized in that** the outlet body (102) further comprises a ball chamber (122) containing a ball-valve (120) collaborating with the pushrod (11) of the support piece (2).

14. Use of a fluid distribution outlet (100) according to either of Claims 12 and 13 for distributing a gas or gaseous mixture, or vacuum within a hospital building.
